# EUROPEAN PATENT APPLICATION

(11) **EP 1 975 250 A1**
(43) Date of publication of application: **01.10.2008**
(21) Application number: 07105005.8
(22) Date of filing: 27.03.2007
(51) Int. Cl.: C12Q 1/68

(54) **Rapid determination of genotypic differences**

(71) Applicant: InPheno AG, 4051 Basel (CH)
(72) Inventor: Hamy, François, 68110 Illzach (FR); Hubert, Stéphane, 68300 Saint-Louis (FR); Klimkait, Thomas, 79539 Lörrach (DE); Vidal, Vincent, 68300 Saint-Louis (FR)
(74) Representative: Becker, Konrad

(57) **Abstract**

The invention is directed to a method for a rapid determination of genetic variants. Such genetic variants are of particular clinical or diagnostic importance if they occur in a disease-related region in the genome of a patient or in microbial or viral pathogens, e.g. in HIV. Heteroduplexes or oligoplexes are formed by incubating the disease-relevant DNA from a biological sample with a corresponding standard DNA probe, separated with capillary electrophoresis, and the composition and amount of the heteroduplexes and oligoplexes measured.

## Description

### Field of the invention

The invention describes a method for rapid determination of genetic variation in a pathogen or in the genome of a patient for diagnostic and/or therapeutic uses.

### Background of the invention

Variations in the genetic material (genotype) of the human immunodeficiency virus (HIV) can dramatically impact phenotypic manifestations such as resistance to treatment, replicative capacity, and host co-receptor usage. Beyond HIV, sequence variations in microbes and viruses may impact the ability to either infect a host, cause different types and grades of pathology, and resistance or susceptibility to therapy.

Several methodologies have been described to characterize genetic information and variations; these include, for example, restriction fragment length polymorphism, sequencing, denaturing HPLC mass-spectrometry (see e.g. Deininger et al., Leukemia 2004, 18, 864-871), gene chips (see e.g. US 4,994,373) and hetero-duplex tracking assay (US 2006/0194227). The usual method for determination of hetero-duplexes are separation techniques using slab gel electrophoresis, which is cumbersome and time-consuming.

### Summary of the invention

The invention relates to a method for analysing disease-relevant DNA derived from a biological sample comprising the steps of
(i) forming a heteroduplex or oligoplex by incubating the disease-relevant DNA with a corresponding standard DNA probe for the disease-relevant genetic region;
(ii) separating different heteroduplexes and oligoplexes formed in step (i) with capillary electrophoresis; and
(iii) measuring the composition and amount of heteroduplexes and oligoplexes.

This method allows the rapid determination of genetic differences in a disease-related region in the genome of a patient afflicted with a disease or within a disease-causing pathogen.

The method may be further combined with extracting nucleic acid (RNA or DNA) from diseased tissue of the patient or from samples of the pathogen, and optionally reverse transcribe RNA into DNA, then amplifying DNA with polymerase chain reaction (PCR). Preferred is such a method applied to DNA or RNA of a microbial or viral pathogen, in particular human immunodeficiency virus (HIV).

For determination of quantitative aspects, formation of homo- and heteroduplexes or oligoplexes may be performed and analysed by capillary electrophoresis.

The invention further relates to a kit for use in such a method.

### Brief description of the figures

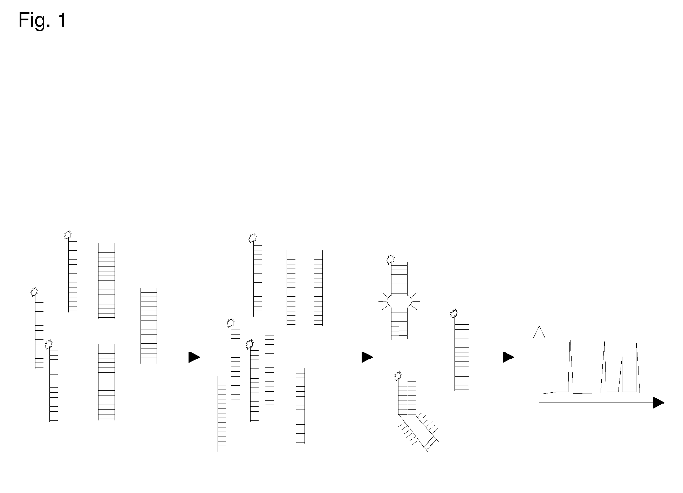

*Figure 1* describes the principle of molecule structures suitable for separation and distinction by the invention. Labelled probes are mixed with double-stranded DNA. Semi-denaturing conditions for duplex formation and separation stabilize the following structures: perfectly matching hybrids (duplex or oligoplex); bulged hybrids due to deletions in one strand; mismatched regions due to sequence differences. Separation on a semi-denaturing capillary matrix results in distinct patterns of peaks that can be assigned and quantified.

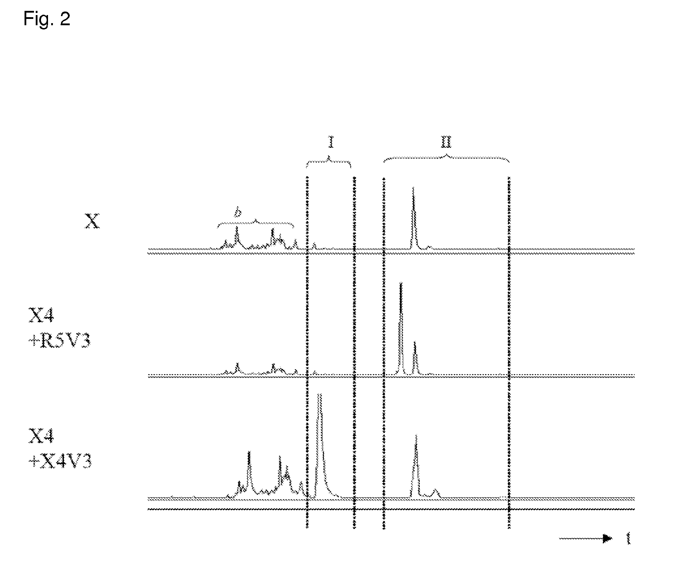

*Figure* 2 exemplifies an analysis using such capillary-based separation. Horizontal line for all graphs as time axis "t". Lane "X4" shows the result of a single stranded probe, "b" summarizing a region with background effects in the matrix; area "I" specifies a time period for homoduplexes, area "II" specifies and distinguishes various heteroduplexed hybdrids. Lanes "X4 + R5V3" and "X4 + X4V3" shows results with mixtures of single stranded probe X4 and double stranded DNA R5V3 and X4V3, respectively.

### Detailed description of the invention

The invention is directed to a method for a rapid determination of genetic variants. Such genetic variants are of particular clinical or diagnostic importance if they occur in a disease-related region of the genome of a patient afflicted with a disease. Furthermore such genetic variants within a pathogen are likewise important for treatment of disease conditions caused by such pathogens, e.g. microbial or viral pathogens, in a patient.

The occurrence and type of genetic variants either in a patient afflicted with a disease or in a microbial or viral pathogen causing a disease in a patient are important for determining (i) treatment susceptibility, (ii) treatment response, or (iii) values for the prediction of treatment outcome and clinical prospects of the patient afflicted with the disease.

Such disease-related region of importance of a patient afflicted with a disease is a region of a gene or gene product. Examples of disease-related regions for viruses are:
Hepatitis C virus: interferon sensitivity determining region (ISDR) NS5A, NS3, NS5B. Hepatitis B virus: reverse transcriptase, core and pre-core region.
Human Immunodeficiency Virus (HIV): Gag, protease, RT, integrase, envelope, TAT.
Transplantation associated polyoma virus, BK and JC viruses: gancyclovir susceptibility region and other drugable regions.
Influenza A and B: hemagglutinin and neuraminidase.

Also in other viruses, drugable regions can be found, for example in Dengue, Ebola, Marburg, Lassa, and West Nile viruses, which cause hemorrhagic fever, and in the Herpes virus family (Herpes simplex virus 1 and 2, cytomegalovirus, human herpes virus 6, varicella zoster virus).

A pathogen, as understood in the context of the present invention, may be a microbial pathogen or pathogen. Examples of microbial pathogens are methycillin resistant Staphylococcus aureus, Neisseria gonorrheae, Chlamidia trachomatis, enteropathogenic Escherichia coli, and Helicobacter pylori.

A patient, as understood in the context of the present invention, is a human or animal patient infected by the pathogen or carrying a disease caused by a genetic variation. In particular such patients are considered for enrolment in a clinical study, or being monitored at onset or during treatment, or at stages of treatment failure or cessation.

The method of the invention comprises the steps of
(a) extracting nucleic acid from diseased tissue or cells of a patient or from a pathogen;
   (a₁) optionally converting RNA of interest by annealing to an oligonucleotide primer, incubating with a reverse transcriptase, and isolating DNA;
(b) amplifying DNA from step (a) or (a₁) with polymerase chain reaction (PCR) using primers selecting a disease-relevant genetic region of the patient or the pathogen;
(c) forming a heteroduplex or oligoplex by incubating the DNA from step (b) with a probe for the disease-relevant genetic region of the patient or of the pathogen;
(d) separating different heteroduplexes and oligoplexes with capillary electrophoresis; and
(e) measuring the composition and amount of heteroduplexes and oligoplexes.

In step (a), nucleic acid of interest is extracted and purified from diseased tissue, cells or pathogen-containing sample. The nucleic acid may be extracted from the diseased tissue or cells of the patient or may be extracted from a microbial or viral pathogen found in tissue or body fluids of the patient. Diseased tissue may be any organ infected by the pathogen, e.g., blood cells, liver, lymph nodes, bowels, kidney, lungs, skin, stomach, testes, or mucosa. Diseased tissue may likewise be tissue carrying a genetic variation in a disease-related region in the genome of a patient, e.g. cancerous tissue. Body fluids considered are blood, e.g. whole blood or fractionated blood or blood serum, saliva, urine, amniotic fluid, or also extracts from feces, cerebrospinal fluid, tears, sputum, bronchoalveolar lavage, semen, or vaginal secretion. The nucleic acids extracted may be DNA or RNA. Methods for extraction are well known in the art. For example a commercial viral nucleic acid preparation kit may be used.

If the genetic material to be analysed is RNA or resembles RNA, then such material is first converted into cDNA in an optional step (a₁). The RNA is annealed to a suitable oligonucleotide primer. The primer is so chosen as to target the RNA of interest. The target nucleic acid of interest can be a gene or a gene sequence in a locus involved in the development of a disease or that is the basis of disease conditions. The target nucleic acid may be a particular gene or gene sequence of an external pathogen found in a diseased tissue or body fluid of the patient, or, for example, a susceptibility gene or a nucleotide sequence of a receptor in the genome of the patient as found in tissue and cells. The gene or gene sequence of interest may encode a target of a selective inhibitor. For example, for HIV the gene sequence is the envelope region targeted by fusion inhibitors. Another example is a gene sequence coding for a cellular molecule targeted by receptor-antagonists.

If the nucleotide to be analysed is DNA, then this optional step (a₁) of reverse transcription can be omitted.

In the step (b) the DNA to be analysed is amplified using standard polymerase chain reaction (PCR). The PCR-driven amplification of the target region is suitable for segments of different sizes, e.g. in the range of 50 base pairs to > 5 kilobase pairs. It is preferable to use two amplification rounds, one to get amplification for DNA in the desired region, but a second round of amplification to generate a DNA fragment of defined short length and with two precisely defined ends. The desired length of the DNA at the end of the amplification is in the range of 50 base pairs to > 5 kilobase pairs, preferably between 100 base pairs and 600 base pairs. Any of the standard procedures known in the art may be used. After amplification the DNA (in the form of double-stranded DNA) is separated from salts, enzyme (polymerase) and primers by standard methods, for example size exclusion chromatography, dialysis, ion-exchange chromatography, filtration, electrophoresis, or precipitation.

In step (c) DNA heteroduplexes or oligoplexes are formed by incubating with a DNA probe for the disease-relevant genetic region of the patient or of the pathogen. A DNA probe complementary to a known target sequence in the genetic material of a pathogen (microbial or viral pathogen) representing a known reference strain of the pathogen to be examined is e.g. synthesized by standard synthetic procedures and terminally labelled with the appropriate label. Likewise a DNA probe complementary to a particular sequence in the genome of the patient may be prepared.

Labels considered are fluorescent labels, for example ROX (5-carboxy-X-rhodamine triethylammonium salt), TAMRA (carboxytetramethylrhodamine), FAM (carboxyfluorescein), Cy3 (cyanine 369) or Cy5 (cyanine 5189), and/or biotin. Alternatively, oligoplexes generated in the hybridization procedure can be labelled by intercalating agents, such as SYBR green or reagents available under the trade name Picogreen.

Particularly preferred is the synthesis of a DNA probe carrying biotin, which allows to purify the probe by chromatographic methods using a stationary phase carrying avidin or streptavidin.

The nucleic acid hybridization occurs between a sequence-specific primer with known sequence and the region of interest in the target nucleic acid from the pathogen or the patient that is to be examined by a heteroduplex or oligoplex formation between the single stranded oligonucleotidic primer and the single stranded version of the target nucleic acid of interest (giving a heteroduplex), or an oligo-plexed version of the oligonucleotide with a single-stranded target gene sequence, or a single stranded oligonucleotide with an oligo-plexed target sequence (giving an oligoplex).

The hybridisation occurs in the present of a suitable salt, for example a mono-ionic salt, for example a potassium salt, such as potassium chloride.

The described duplex-/oligoplex-formation of DNA strands from different origin, one strand from the labelled probe and the second from the sample under investigation, occurs thereby under carefully selected constant conditions. Preferred conditions are semi-denaturing conditions in the presence of a concentration of 0.5 to 10 mM mono-ionic salt, e.g. 1 to 5 mM potassium salt. Typical preferred conditions are, for example, controlled pH between 7.0 and 8.0, controlled temperature between 18 and 28°C, mono-ionic salts such as KCI, in a concentration of between 1 and 10 mM, quantity of target and probe in the region between 10 and 20 ng and 2 ng, respectively, and an aqueous solvent.

In step (d) capillary electrophoresis is performed with a standard commercial matrix, in particular a semi-denaturing matrix. In particular, the standard matrix has sieving capability separating nucleic acid complexes of the required molecular weight range, for example a conformational analysis polymer as available from Applied Biosystems. Suitable buffers are Tris-borate, Tris-acetate, MOPS, mixtures thereof, or other buffering systems that allow the establishment of semi-denaturing conditions for nucleic acids, which facilitate a clear separation of nucleic acid complexes and oligo-plexes based on their physical properties, i.e. the structure or shape based, perfect or imperfect nucleic acid-nucleic acid hybrids. The presence of a mono-ionic salt, e.g. a potassium salt in a concentration of 0.5 to 10 mM, is preferred. Other crucial conditions include the use of defined temperature profiles between 18°C and 30°C and the means to keep temperatures constant throughout the analysis. These ensure that DNA-DNA-hybrids (duplex or oligoplex) can be separated and distinguished from each other. For capillary electrophoresis standard equipment may be used. Changes in voltage can be used to modulate peak resolution and time to or precision of results. Likewise variations in temperature (to be kept constant throughout the run) allows to modulate peak resolution according to the needs of the application.

In step (e) the composition and amount of heteroduplexes and oligoplexes is measured. Preferably the duplexes and oligoplexes are detected by fluorescence emission. As a result, distinct duplexes and oligoplexes are displayed on an electropherogram where fluorescence intensity is plotted as a function of migration time. In a series of validation experiments defined respective migration zones for the possible homotropic and heterotropic duplexes and oligoplexes are determined. The method then allows a quantification of the signal in the respective zones formed when a sample from a patient is analysed.

The results of the analysis may be translated into predicted phenotypic behaviour, e.g., for the HIV envelope region, into viral tropism.

As can be seen from the description of the steps of the method as used in the invention, the steps (a), (a1) and (b) are standard procedures in the isolation and further manipulation of nucleotides. Likewise the analysis of a genetic variation by determination of the composition of the DNA on isolation and determination of heteroduplexes or oligoplexes represents a standard procedure well known in the art. The invention therefore resides in particular in a method comprising the steps of
(i) forming a heteroduplex or oligoplex by incubating a disease-relevant DNA derived from a biological sample with a corresponding standard DNA probe for the disease-relevant genetic region;
(ii) separating different heteroduplexes and oligoplexes formed in step (i) with capillary electrophoresis; and
(iii) measuring the composition and amount of heteroduplexes and oligoplexes.

A particular aspect of the invention is the use of a capillary-based methodology that enables a standardized and/or high throughput processing of the materials for analysis and of the molecular diagnostic test itself. A micro-capillary may be used preferably, yet alternative methodologies and matrices and other systems amenable for separation of nucleic acids based on physical methods and other than the standard slab gels are foreseen that can principally be applied, and such methods are likewise included into the invention.

In the context of the present invention, high throughput means an optimised procedure for parallel analysis superior to individual sample handling. Appropriate format handles 8, 16, to 96 samples at a time.

Current methodologies, which are based upon non-denaturing slab gels, bear as one inherent limitation their 2-D geometry that links any scale-up to a linear increase in needed surface area of the gel. The innovative step to using capillaries, thereby, is far from trivial as conditions dictated by geometry, surface effects, charge effects and running conditions (buffer, temperature, electrical field) of such format could neither be extrapolated nor predicted from the slab format nor could even the feasibility of such alteration be foreseen.

The results of the inventive method may be used (i) for drug discovery, where activity and potency of new treatment modalities can be assessed *in vitro* based on results from analyses emerging from an application of the invention, in particular where known genetic alterations can be linked to an expected treatment success; (ii) for screening of patients prior to, enrolment in, and monitoring during clinical studies by analysing patient-derived material by said methodology in a modification specific for the application; and (iii) for diagnostic use accompanying treatment in routine settings.

The nucleic acid duplex (or oligoplex) formation between the known single stranded probe and the sample DNA occurs in a strictly stoichiometric fashion. By applying defined, known concentrations of the probe this process enables the quantification of hetero-plex *versus* homo-plex *versus* free probe in any given sample reaction. This then allows to define in mixed populations of sequences in the material to be examined the relative fraction of any respective unknown nucleotide sequence. Such application is of particular use in diseases or conditions with mixed populations of the target gene or gene product, e.g. simultaneous infections wit pathogenic *versus* non-pathogenic variants of the same pathogen, susceptible *versus* drug-resistant versions of a pathogen, or aberrant *versus* normal version of a cell in diseased tissue, for example cancer tissue.

In particular the invention concerns a method comprising the steps of
(i) forming a duplex or oligoplex by incubating a known amount of a DNA mixture comprising disease-relevant variant DNA and standard DNA derived from a biological sample with a corresponding standard DNA probe for the disease-relevant genetic region;
(ii) separating different duplexes and oligoplexes formed in step (i) with capillary electrophoresis; and
(iii) measuring the composition and amount of duplexes and oligoplexes.

A particular example wherein such quantitative method may be used is in analysis of HIV infection: On various defined mixtures or on inherently mixed HIV populations the respective hybridization reactions generate distinct products for (i) free probe, (ii) homoduplex (2 sequences conferring identical tropism), and (iii) heteroduplex (2 sequences conferring different tropism). The corresponding areas under the curves, expressed as per cent of the total reaction, correlate well with the fraction of the respective product in the deliberate mixtures. Material from similarly heterogeneous sequence populations in the patient sample enable a quantitative assessment e.g. (i) of the earliest detection of potentially dangerous sequences (as they are the basis for the expression of the pathogenic principle), (ii) of a trend of development of such sequences over time (does a treatment lead to a diminution or can an exacerbation be correlated with an increase of such pathogenic sequences as evidence for the amplification of an organism, virus, or cells with pathogenic potential), or (iii) of the emergence of new, e.g. resistant forms of sequence-carrying pathogens during treatment or after cessation of treatment. Further application of the principle described in this invention can be developed by the skilled person in the art in the diagnosis and treatment of relevant diseases.

A special application of the method of the invention, e.g. for quantification during patient screening or monitoring, may occur subsequent to the application of a standard phenotyping method with sensitive detection: For example the quantity of supernatant virus (of a pathogenic virus) produced in a fixed time window and determined by a phenotyping method with appropriate sensitivity is directly subjected to the quantitative examination according to the present invention. This test assesses the genetic representation of the virus in a clinical sample. The test can serve as stand-alone analysis or be used as complementing function in combination with a phenotyping method.

Such standard phenotyping methods are well known in the art. A preferred phenotypic method for HIV to be combined with the method of the invention described here is the procedure and kit known under the trademark PhenX-R (InPheno, Basel, Switzerland).

The invention further concerns a kit suitable for the performance of the inventive method. A kit according to the invention comprises ingredients necessary to successfully conduct an analysis, such as (i) required solutions and buffers for preparing the sample from biological material (blood, tissue, cell culture materials); (ii) oligonucleotide primers (probes), reaction buffer, control reference compounds and enzymes for PCR amplification; (iii) sample preparation solution for loading onto capillary according to the capillary system used and with recommendations depending on the system applied; (iv) all information on appropriate running conditions (including capillary dimension, matrix, voltage, temperature) for successful conduct of the analysis; (v) positive control to be run on the capillary for reference.

The minimum kit according to the invention will comprise (a) oligonucleotide primers; (b) sample preparation solution for loading onto capillary according to the capillary system used and with recommendations depending on the system applied; (c) all information on appropriate running conditions (including capillary dimension, matrix, voltage, temperature) for successful conduct of the analysis; (d) positive control to be run on the capillary for reference. Such a minimum kit may be combined with other kits known for sample isolation and PCR.

The kit will have to be adapted to the particular case to be analysed, allowing pre-formulation of all ingredients and may be a complete test system to be used on standard laboratory equipment.

### Example

### Nucleic acid extraction

HIV RNA is extracted from plasma of a patient using a commercial viral nucleic acid preparation kit (Nucleospin RNA-virus, Macherey-Nagel, Switzerland) according to the manufacturer's protocol. RNA is finally eluted into 40 µL Tris/EDTA 10 mM / 1 mM, (pH 7.2) and stored aliquoted at -80°C until use. If DNA is extracted, this is eluted into the same mixture, but at a pH of 8.0, and also stored at-80°C.

### cDNA conversion

The primer Env-RT3 (tcccactccatccaggtcat, SEQ ID NO:1) is added to the extracted RNA, and the mixture heated at 70°C for 3 minutes and cooled to room temperature just prior to reverse transcription. Reverse transcription into DNA is initiated by addition of 200 units of Stratascript 5.0 (Stratagene) in a final volume of 20 µL containing 1 mM dNTPs, 1 unit RNase inhibitor and 10 mM di-thiothreitol. Reverse transcription is performed by incubation at 42°C for 35 minutes and subsequently at 55°C for 25 minutes, and the enzyme is then heat-inactivated at 70 °C for 15 minutes. The type of reverse transcriptase used in the example is not critical, yet Stratascript has been found to work well.

### First amplification

The HIV target region within the envelope spanning from nucleotides 6846 to 8118 (according to the numbering of HIV-1 reference virus NL4-3, Genbank accession number AF324493) is amplified by polymerase chain reaction. Each amplification reaction contains 10 µL of cDNA, 0.5 units of thermostable, proofreading DNA polymerase (i-Proof, Biorad), 10 pmole of forward primer D_6846 (aggcctgtccaaaggtatcctttga, SEQ ID NO:2) and 10 pmoles of reverse primer Env-RT3 (SEQ ID NO:1), dNTPs, and reaction buffer (as provided by vendor). Following amplification for 30 cycles, the obtained amplicon (1,287 bp) is retrieved for further utilization as template for the generation of DNA fragments that are amenable to use in hybridization-based analysis.

### Second amplification

Using the amplification product obtained from the first amplification (see above) a nested PCR is performed in order to generate a 141 bp DNA fragment encompassing the variable region 3 (V3) within the HIV envelope gene gp120 (nucleotides 7092 to 7232; numbering according to NL4-3 sequence, Genbank accession number AF324493). For this, 2 µL of the reaction mixture from the first amplification is reamplified in a 50 µL final volume reaction containing 10 pmoles of forward primer V3_Fwd (gaatctgtagaaattaattgtacaagac, SEQ ID NO:3), 10 pmoles of reverse primer V3_Rev (tgctctactaatgttacaatgtgcttgtcttat, SEQ ID NO:4), 0.5 units of thermostable, proofreading DNA polymerase (i-Proof, Biorad), dNTPs and buffer. Following 30 rounds of amplification, the presence, apparent purity and approximate size of second amplicon are verified by migrating 10% of the PCR product through a 2% agarose gel. The remaining material of the polymerase chain reaction (PCR) product is separated from salts, enzyme and primers using size exclusion chromatography on a Microcon column (Millipore, MW cut-off: 100 kDa) to yield the double-stranded DNA fragment "Target-V3". The concentration of the purified DNA was determined using a NanoDrop ND-1 000 spectrophotometer.

### Synthesis of the probe

Starting from laboratory-adapted strains representing CXCR4- and CCR5-tropic viruses such as HXB2 (Genbank accession number NC_001802) and JR-CSF (Genbank accession number AY_426126) the V3 loop region is amplified by PCR using biotin-labelled V3-Fwd and V3-Rev primers, which have been fluorescently labelled with ROX (5-carboxy-X-rhodamine triethyl ammonium salt) or FAM (carboxyfluorescein). These biotinylated PCR products are then purified following binding to streptavidin-coated magnetic beads. A pure single-stranded fluorescent probe is then recovered by alkali denaturation of the double-stranded PCR product, which is bound to the beads via the biotin-modified second strand. Following neutralisation with a 1 M Tris-HCl pH 8.0 solution, the quantity of single-stranded DNA probe obtained is determined spectrophotometrically. By means of this binding reaction, pure single stranded products can be produced for the subsequent oligoplex formation.

### Generation of DNA oligoplexes and separation on capillary electrophoresis

Hetero- or homoduplexes are generated following mixing of probe and "Target-V3" at their optimal concentrations, 2 ng and 15 - 20 ng, respectively, in the presence of 1 mM KCI As buffer and for nucleic acid stabilization 1 mM Tris-Cl pH 8.0, and 0.1 mM EDTA are used. After heat-denaturation at 95°C the reaction is chilled by rapid transfer onto ice. Heteroduplexed DNA segments are then resolved by capillary electrophoresis on an ABI310 capillary gene-analyser (Applied Biosystems) in a conformational analysis polymer matrix made 5% in 1 mM EDTA, 10% glycerol, 1 x TBE 10% glycerol as electrophoresis buffer, at a run voltage of 6 kilovolts at a constant temperature of 30°C. The different oligoplexes are separated during electrophoresis and detected by fluorescence emission. As a result, distinct oligoplexes are displayed on an electropherogram where fluorescence intensity is plotted as a function of migration time.

## Claims

1. A method for analysing disease-relevant DNA derived from a biological sample comprising the steps of
(i) forming a heteroduplex or oligoplex by incubating the disease-relevant DNA with a corresponding standard DNA probe for the disease-relevant genetic region;
(ii) separating different heteroduplexes and oligoplexes formed in step (i) with capillary electrophoresis; and
(iii) measuring the composition and amount of heteroduplexes and oligoplexes.

2. The method according to claim 1 for the rapid determination of genetic differences in a disease-related region in the genome of a patient afflicted with a disease or within a disease-causing pathogen comprising the steps of
(a) extracting nucleic acid from diseased tissue of the patient or from samples of the pathogen;
(a1) optionally converting RNA of interest by annealing to an oligonucleotide primer, incubating with a reverse transcriptase, and isolating DNA;
(b) amplifying DNA from step (a) or (a1) with polymerase chain reaction (PCR) using primers selecting a disease-relevant genetic region of the patient or the pathogen;
(c) forming a heteroduplex or oligoplex by incubating the DNA from step (b) with a probe for the disease-relevant genetic region of the patient or of the pathogen;
(d) separating different heteroduplexes and oligoplexes with capillary electrophoresis; and
(e) measuring the composition and amount of heteroduplexes and oligoplexes.

3. The method according to claim 2 wherein the nucleic acid in step (a) is DNA from a microbial pathogen.

4. The method according to claim 2 wherein the nucleic acid in step (a) is RNA from a microbial pathogen.

5. The method according to claim 2 wherein the nucleic acid in step (a) is DNA from a viral pathogen.

6. The method according to claim 2 wherein the nucleic acid in step (a) is RNA from a viral pathogen.

7. The method according to claim 6 wherein the nucleic acid in step (a) is RNA from HIV.

8. The method according to claim 1 or 2 wherein the DNA forming a heteroduplex or oligoplex is double-stranded DNA of between 100 and 500 base pairs.

9. The method according to claim 1 or 2 wherein in the heteroduplex or oligoplex forming step and the capillary electrophoresis semi-denaturing conditions are chosen.

10. The method according to claim 9 wherein the heteroduplex or oligoplex forming step and the capillary electrophoresis are performed in the presence of 0.5 to 10 mM potassium chloride.

11. A method for analysing disease-relevant DNA derived from a biological sample comprising the steps of
(i) forming a duplex or oligoplex by incubating a known amount of a DNA mixture comprising disease-relevant variant DNA and standard DNA derived from a biological sample with a corresponding standard DNA probe for the disease-relevant genetic region;
(ii) separating different duplexes and oligoplexes formed in step (i) with capillary electrophoresis; and
(iii) measuring the composition and amount of duplexes and oligoplexes.

12. The method according to claim 1 or 11 for high throughput processing of patient tissue and pathogen samples.

13. A kit for analysing disease-relevant DNA derived from a biological sample comprising
(a) oligonucleotide primers;
(b) sample preparation solution for loading onto capillary according to the capillary system used and with recommendations depending on the system applied;
(c) all information on appropriate running conditions for successful conduct of the analysis; and
(d) positive control to be run on the capillary for reference.

14. The kit according to claim 13 further comprising
(i) required solutions and buffers for preparing the nucleotide sample from biological material; and
(ii) reaction buffer, control reference compounds and enzymes for PCR amplification.


SEQUENCE LISTING

<110>  InPheno AG

<120>  Rapid determination of genotypic differences

<130>  P350

<160>  4

<170>  PatentIn version 3.3

<210>  1
<211>  20
<212>  DNA
<213>  Artificial sequence

<220>
<223>  primer Env-RT3

<400>  1
tcccactcca tccaggtcat                                                    20

<210>  2
<211>  25
<212>  DNA
<213>  Artificial sequence

<220>
<223>  primer D_6846

<400>  2
aggcctgtcc aaaggtatcc tttga                                              25

<210>  3
<211>  28
<212>  DNA
<213>  Artificial sequence

<220>
<223>  primer V3_Fwd

<400>  3
gaatctgtag aaattaattg tacaagac                                           28

<210>  4
<211>  33
<212>  DNA
<213>  Artificial sequence

<220>
<223>  primer V3_Rev

<400>  4
tgctctacta atgttacaat gtgcttgtct tat                                     33